# EUROPEAN PATENT APPLICATION

(11) **EP 2 477 031 A2**
(43) Date of publication of application: **18.07.2012**
(21) Application number: 10815570.6
(22) Date of filing: 27.08.2010
(51) Int. Cl.: G01N 33/68, G01N 33/50

(54) **METHOD FOR DETECTING AND QUANTIFYING A TARGET SUBSTANCE USING A BIOCHIP**

(30) Priority: 09.09.2009 KR 20090085091
(71) Applicant: Korea Research Institute of Bioscience and Biotechnology, Yuseong-gu, Daejeon 305-333 (KR)
(72) Inventor: CHUNG, Bong Hyun, Daejeon 302-280 (KR); KIM, Sang Kyu, Taean-gun Chungcheongnam-do 357-944 (KR); CHO, Hyun Min, Changwon-si Gyeongsangnam-do 641-200 (KR)
(74) Representative: Mai, Dörr, Besier
(86) International application number: PCT/KR2010/005785
(87) International publication number: WO 2011/031025

(57) **Abstract**

The present invention relates to a method for detecting and quantifying a target substance using a biochip having a substrate onto which probe molecules are fixed, and more particularly, to a method for detecting and quantifying a target substance with the naked eye by using a biochip, comprising the steps of preparing a biochip having a substrate onto which probe molecules are fixed, contacting the biochip with a sample containing a target substance having electric charges, reacting the target substance with nanoparticles having electric charges that are opposite to those of the target substance, and then reacting with a metal enhancing solution so as to amplify the size of the nanoparticles.

## Description

### [Technical Field]

The present invention relates to a method for detecting and quantifying a target substance using a biochip having a substrate onto which probe molecules are fixed, and more particularly, to a method for detecting and quantifying a target substance with the naked eye by using a biochip, comprising the steps of preparing a biochip having a substrate onto which probe molecules are fixed, contacting the biochip with a sample containing a target substance having electric charges, reacting the target substance with nanoparticles having electric charges that are opposite to those of the target substance, and then reacting with a metal enhancing solution so as to amplify the size of the nanoparticles.

### [Background Art]

A biochip is a hybrid device made of an existing semiconductor type chip by combining bio-organic materials isolated from creatures, such as enzymes, proteins, antibodies, DNA, microbes, animal/plant cells and organs, or animal/plant neurons and organs, with inorganic matters such as semiconductors or glass. The biochip acts to diagnose infectious diseases or analyze genes by using inherent functions to biomolecules and mimicking functions of organisms, and acts as a new functional device for processing new information.

According to biomaterials and systemization, biochips can be classified into a DNA chip, an RNA chip, a protein chip, a cell chip, and a neuron chip. Also, in a broad definition, biochips include a lab chip having automatic analysis functions including pretreatment of samples, biochemical reaction, detection, and data analysis, and a biosensor having detection and analysis functions of various biochemical materials.

In addition, biochips can be largely divided into two types, depending on the method of detection of the biomaterials; one being a microarray type, which detects a specific biochemical material contained in a sample using capturing probes. Specifically, a substance that is able to function as a capturing probe is fixed onto the chip surface, and then a biochemical material to be analyzed is reacted thereto, and its reaction is detected and analyzed, thereby obtaining the information on the biochemical material. The other type is a microfluidic biochip, in which a microchannel, a microchamber, and a mixer valve are provided on the chip to control a microfluid, a biochemical material is immobilized onto a detecting unit, and then a biochemical material to be analyzed is reacted with the biochemical material immobilized onto the detecting unit by microfluidic flow so as to detect its reaction. This field has been the most actively studied with the recent miniaturization trends and when taking long-term point of view.

Fabrication and utilization of a biochip requires a probe immobilization technique capable of reacting with a reaction material, a detection technique capable of detecting the reaction, and an information processing technique capable of processing the detected information.

Among these techniques, the reaction detection technique is generally performed using a detectable label such as fluorescent and colorimetric labels, and isotopes. The labeling is important in increasing detection sensitivity. However, it is problematic, as biomolecules can be modified by labeling, or low-molecular weight-materials cannot be labeled. There are also problems in that the labeling process requires a large amount of sample and should undergo 2-3 further steps, and labeling difference between various proteins increases quantification errors. Laser-induced fluorescence detection method is the current representative labeling method. Laser-induced fluorescence detection method is the most popular optical method, in which a sample is labeled with a fluorescent material and its reaction with probes immobilized onto the substrate is detected using the labeled fluorescent material. However, this method is also problematic in that a needed optical measurement system for detecting the reaction is needed to require much time and costs, and this detection method also has a limitation in miniaturization of a biochip-based analysis system. Further, an additional step of labeling target molecules in the sample with a fluorescent material is needed, and thus it is inconvenient compared to label-free detection methods.

Accordingly, the demand for label-free detection is increasing in the biochip technology field. One of the label-free detection methods is an electrochemical detection method which detects the electrochemical reaction of chemical materials with a sample that is applied to the probe-modified electrode, but the method has a problem of relatively low sensitivity, compared to the fluorescence detection methods.

As such, the conventional detection methods have a limitation in the efficiency, and thus always involve problems concerning the lowered reliability and satisfaction in their application to biochemical practice.

### [Disclosure]

### [Technical Problem]

The present inventors have made many efforts to overcome the problems of requiring additional expensive equipment, and of the inefficiency of conventional biochips. As a result, they found that when metal nanoparticles are reacted with a target DNA binding with a probe, and a metal enhancing solution is reacted thereto, the metal nanoparticles are amplified by the metal enhancing solution to be detectable with the naked eye, thereby performing label-free detection without additional equipment and quantifying the target substance using a general scanner.

### [Technical Solution]

An object of the present invention is to provide a method for detecting a target substance using a biochip, comprising the steps of: (a) fixing probe molecules onto a substrate; (b) contacting the substrate, onto which probe molecules are fixed, with a sample containing the target substance having electric charges; (c) reacting the target substance with nanoparticles having electric charges that are opposite to those of the target substance; and (d) reacting with a metal enhancing solution.

Another object of the present invention is to provide a method for quantifying a target substance using a biochip, comprising the steps of: (a) fixing probe molecules onto a substrate; (b) contacting the substrate, onto which probe molecules are fixed, with a sample containing the target substance having electric charges; (c) reacting the target substance with nanoparticles having electric charges that are opposite to those of the target substance; (d) reacting with a metal enhancing solution; and (e) measuring the reaction intensity of the region that reacts with the metal enhancing solution.

### [Advantageous Effects]

The method for detecting a target substance using a biochip of the present invention allows direct observation with the naked eye and quantification analysis using a simple scanner, unlike the conventional fluorescence detection methods requiring a process of fluorescence labeling and expensive equipment.

### [Description of Drawings]

FIG. 1 is a schematic view showing the fabrication and analysis principle of a biochip according to the present invention;
FIG. 2 is a diagram showing the measurement of hybridized DNA using a general fluorescence;
FIG. 3 is a diagram showing the measurement of hybridized DNA according to the present invention using an optical scanner, in which (a) shows the reaction of 100 nM H5 DNA and (b) shows the reaction of 100 nM HM DNA; and
FIG. 4 is a diagram showing changes in grayscale values according to the DNA concentration using a metal enhancing method of the present invention.

### [Best Mode]

In one embodiment to achieve the above objects, the present invention relates to a method for detecting a target substance using a biochip, comprising the steps of: (a) fixing probe molecules onto a substrate; (b) contacting the substrate, onto which probe molecules are fixed, with a sample containing the target substance having electric charges; (c) reacting the target substance with nanoparticles having electric charges that are opposite to those of the target substance; and (d) reacting with a metal enhancing solution.

As used herein, the term "biochip" generally means integration of high-density microarrays of biomaterials such as DNAs, proteins, and cells onto a variety of surface-modified solids including high molecular substrates such as glass, silicon, and polypropylene.

In step (a) of fixing probes onto the substrate, the type of substrate is not particularly limited, as long as it is a solid substrate typically used in the art for the fabrication of biochips. The substrate may be preferably glass, alumina, ceramic, carbon, gold, silver, copper, aluminum, compound semiconductor and silicon, and most preferably a glass substrate. The substrate is surface-modified, and the surface-modification is performed in order to facilitate attachment and immobilization of the probe molecules. In addition, the surface-modification may be performed in order to include functional groups for the immobilization of biomolecules onto the substrate surface of the biochip. For example, the substrate may be surface-modified with an aldehyde group, a carboxyl group, or an amine group. A glass substrate or a semiconductor substrate may be treated with silane to form an amino group (-NH3, - NH2, etc.). For effective silane treatment, a treatment for preparation of hydroxyl group (-OH) may be performed before silane application. With respect to the objects of the present invention, the immobilization method of the probe molecules onto the substrate is not particularly limited, and a chemical or physical method may be used.

As used herein, the term "probe" refers to a substance capable of specifically binding with a target substance to be analyzed in a sample, and refers to a substance capable of specifically detecting the presence of the target substance in the sample through binding. As the probe molecule, any probe typically used in the art may be used without limitation, and preferably may be PNA (peptide nucleic acid), LNA (locked nucleic acid), peptide, polypeptide, protein, RNA or DNA, and is most preferably PNA. More specifically, the probe may include biomolecules derived from organisms or analogs thereof, or ex vivo generation, and exemplified by enzymes, proteins, antibodies, microbes, animal/plant cells and organs, neurons, DNA, and RNA, in which DNA includes cDNA, genomic DNA, and oligonucleotide, RNA includes genomic RNA, mRNA, and oligonucleotide, and protein includes antibody, antigen, enzyme, and peptide.

In one specific embodiment of the present invention, the surface of a glass substrate is treated with O₂ plasma to expose -OH groups on the surface of the glass substrate, and amine functionalization of the surface is performed, and the amine-treated surface is replaced with carboxyl groups, followed by immobilization of PNA, which is a probe having an amine group (-NH₂), onto the substrate via a covalent peptide bond. A substrate with no PNA is blocked by reacting with PEG (polyethylene glycol) having an amine group in order to avoid background staining (Examples 1 and 2).

Step (b) of contacting the substrate, onto which probe molecules are fixed, with a sample containing the target substance having electric charges is a contacting step for detecting the target substance capable of specifically binding with the probe molecules.

As used herein, the term "target substance" refers to a substance, the presence of which in the sample is detected using a biochip. As the target substance, any substance typically used in the art may be used without limitation, and preferably may be PNA (peptide nucleic acid), LNA (locked nucleic acid), peptide, polypeptide, protein, RNA or DNA, and is most preferably DNA. More specifically, the target substance may include biomolecules derived from organisms or analogs thereof, or ex vivo generation, and exemplified by enzymes, proteins, antibodies, microbes, animal/plant cells and organs, neurons, DNA, and RNA, in which DNA includes cDNA, genomic DNA, and oligonucleotide, RNA includes genomic RNA, mRNA, and oligonucleotide, and protein includes antibody, antigen, enzyme, and peptide.

The target substance has a negative electric charge or a positive electric charge, and the electric charge is opposite to that of the nanoparticles to be reacted. Thus, it is able to bind with nanoparticles by electrostatic attraction.

As used herein, the term "sample" includes tissues, cells, whole blood, serum, plasma, saliva, phlegm, cerebrospinal fluid or urine containing the target substance to be detected, but is not limited thereto.

When the sample containing the target substance is contacted with the substrate onto which probe molecules are fixed, the target substance in the sample specifically binds with the probe molecule. If the target is PNA, LNA, RNA or DNA, specific hybridization between complementary sequences occurs. If the target is a protein, a complex formation may occur by direct binding between the sample protein and the probe, or binding including deformation or modification of the probe by enzymatic reaction of the sample protein may occur. Therefore, the binding reaction between the target protein and the probe molecule may include binding reactions between proteins and biomolecules such as an antigen-antibody reaction and ligand-receptor reaction, or substrate-enzyme reaction between proteins and biomolecules.

In one specific embodiment of the present invention, a specific hybridization is performed by contacting PNA fixed on the substrate with a sample containing a target DNA having a sequence complementary thereto (Example 3).

Step (c) of reacting the target substance with nanoparticles having electric charges that are opposite to those of the target substance is a step of reacting and binding nanoparticles with the target substance specifically binding with the probe.

The nanoparticles are nanoparticles having electric charges that are opposite to those of the target substance, and any nanoparticles typically used in the art may be used without limitation. The nanoparticles may be preferably gold, silver, or platinum, and most preferably gold nanoparticles.

In one preferred embodiment, reaction of the nanoparticles having electric charges that are opposite to those of the target substance may be performed by electrostatic binding.

In one specific embodiment of the present invention, the probe PNA capable of detecting a target DNA was fixed on the substrate, and the region that does not react with the probe PNA was blocked using a blocking molecule to reduce non-specific reaction. When the target DNA to be analyzed is reacted thereto, it specifically reacts with the probe PNA having a complementary sequence, and the surface of the chip becomes negatively charged by the negative electric charges of the phosphate groups of DNA. In this regard, PNA that does not react with DNA do not have negative electric charges, and thus the surface has no electric charges. After reacting with the target DNA, when metal nanoparticles having positive electric charges are reacted thereto, nanoparticles bind to DNA specifically binding with PNA by electrostatic binding, and nanoparticles do not bind to the region where no DNA hybridization occurs.

In one preferred embodiment, reaction of the nanoparticles having electric charges that are opposite to those of the target substance may be performed by biological binding.

In one preferred embodiment, reaction of the nanoparticles having electric charges that are opposite to those of the target substance may be performed by chemical binding.

Step (d) of reacting with a metal enhancing solution is a step of amplifying the size of the nanoparticles binding to the target substance that specifically binds with the probe, and detecting the target substance with the naked eye.

As used herein, the term "metal enhancing solution" means a solution composed of metal ions, which amplifies the size of the nanoparticles by reduction of metal ions surrounding the metal nanoparticles that are used as a catalyst. Any metal enhancing solution typically used in the art may be used without limitation, as long as it is able to amplify the size of the nanoparticles. The metal enhancing solution may be preferably a solution containing gold (Au), silver (Ag), copper (Cu), platinum (Pt) or palladium (Pd) ions, and most preferably a solution containing gold ions.

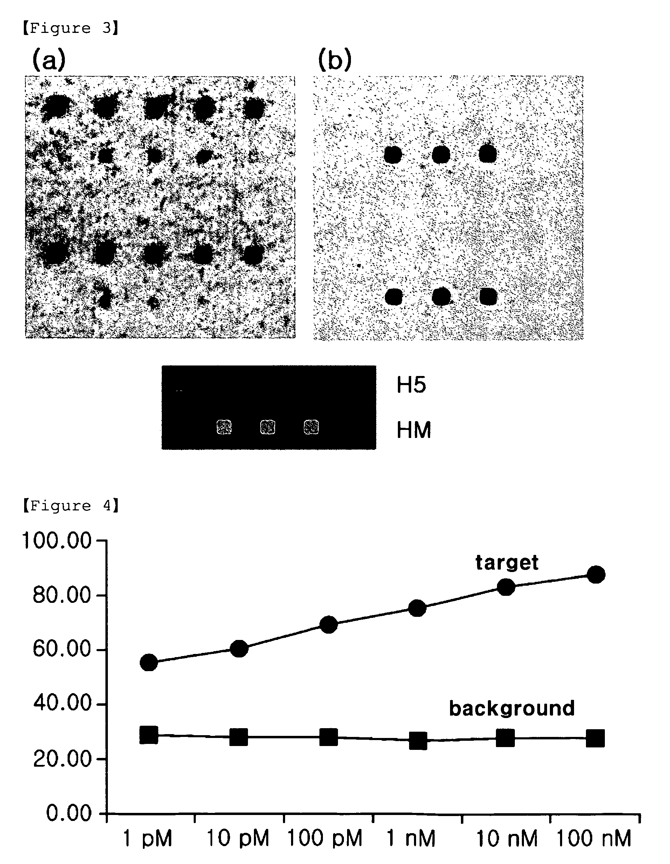

In one specific embodiment of the present invention, 5 nm gold nanoparticles having positive electric charges were reacted, and a gold enhancing solution was reacted for 1 minute. As a result, gold nanoparticles were surrounded by the metals due to reduction of gold ions, and thus the particle size increased. Consequently, the region binding with DNA showed a gray-color, and could be observed with the naked eye. Specifically, the region specifically binding with PNA showed a dark gray color, and the non-specific region showed a very weak gray color or no color (FIG. 3). According to the method of the present invention, the target substance can be simply detected with the naked eye by using a biochip without labeling the target substance or probe molecule with fluorescent materials or without additional optical system or fluorescence scanner.

In another embodiment, the present invention relates to a method for quantifying a target substance using a biochip, comprising the steps of: (a) fixing probe molecules onto a substrate; (b) contacting the substrate, onto which probe molecules are fixed, with a sample containing the target substance having electric charges; (c) reacting the target substance with nanoparticles having electric charges that are opposite to those of the target substance; (d) reacting with a metal enhancing solution; and (e) measuring the reaction intensity of the region that reacts with the metal enhancing solution.

Steps (a) to (d) are the same as the above described.

Step (e) of measuring the reaction intensity of the region that reacts to the metal enhancing solution is a step for quantifying the target substance present in the sample.

As the concentration of the target substance present in the sample increases, the reaction intensity of the region reacted to the metal enhancing solution increases. Therefore, the target substance can be quantified, and analyzed by using a general optical scanner.

In one specific embodiment of the present invention, it was found that the gray spots became deeper and larger, as the concentration of the target DNA increased from 1 pM to 100 nM. Further, they were analyzed as grayscale using a general scanner and Adobe Photoshop software. As a result, as the concentration of the target DNA increased, the grayscale values increased. On the contrary, the constant values were observed in the surrounding substrate that did not bind with the target DNA, irrespective of the concentration (FIG. 4). Thus, it is possible to perform quantification of the target DNA using a general scanner and software.

### [Mode for Invention]

Hereinafter, the present invention will be described in more detail with reference to Examples. However, these Examples are for illustrative purposes only, and the invention is not intended to be limited thereby.

### Example 1: Treatment of Glass Substrate

A glass substrate was washed using a piranha solution, and treated with O₂ plasma to expose -OH groups on the surface of the glass substrate. The glass substrate was reacted with 2% APTES (aminopropyltriethoxysilane) prepared in an ethanol solution for 2 hours. After 2-hr reaction, the surface was washed with ethanol, dried, and then baked on a 120°C hot plate for 1 hour so as to functionalize the surface of the glass substrate with amine. The substrate was further modified by overnight immersion in a solution of 1 M succinic anhydride in DMF (dimethylformamide) solvent, and subsequently washed so as to replace the amines of the glass substrate with carboxyl groups (-COOH).

### Example 2: Fabrication of Biochip

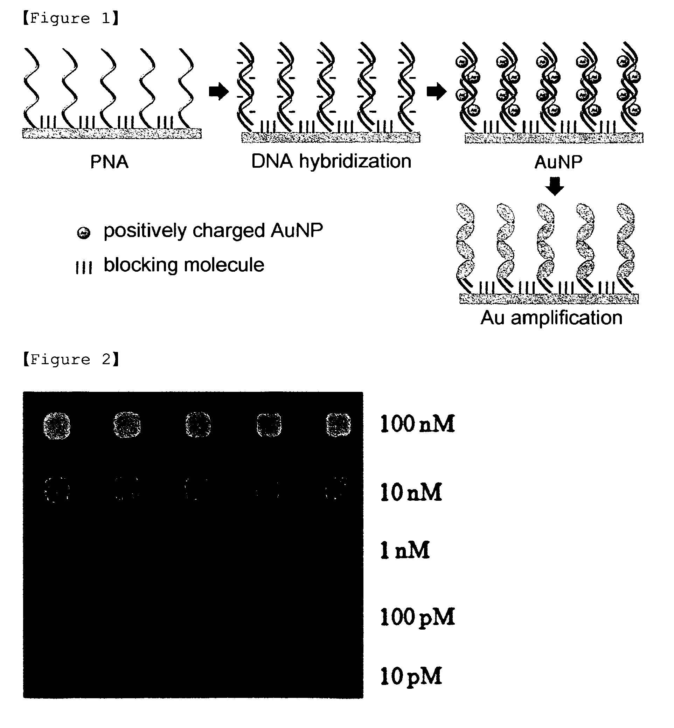

A chip was fabricated in the order as in FIG. 1. First, EDC/NHS was reacted for 15 minutes, and then 50 µM of PNA (peptide nucleic acid, Panagene, Korea) consisting of NH₂-O-AATGGTTTATTCTGCTCA (hereinbelow, referred to as "H5") and 50 µM of control PNA consisting of NH₂-O-GACATCAAGCAGCCATC (hereinbelow, referred to as "HM") were reacted for 1 hour to fix the probe PNA onto the glass substrate prepared in Example 1. In order to block the region onto which no PNA was fixed, 1 mM PEG (polyethylene glycol) having amine at end was reacted for 1 hour to fabricate a probe-attached biochip.

### Example 3: Specific Hybridization with Target DNA

In order to confirm the specific hybridization between the probe-attached biochip fabricated in Example 2 and a target DNA, the target DNA (Bioneer, Korea) having SEQ ID NO. 1 of TGA GCA GAA TAA ACC ATT being complementary to the probe H5 PNA and a control group having SEQ ID NO. 2 of GAT GGC TGC TTG ATG TC were diluted in PBST for hybridization at each concentration.

### Example 4: Amplification and Measurement of Specific Hybridization with Target DNA

For amplification and measurement of specific hybridization with the target DNA, the biochip hybridized in Example 3 was washed with PBST and PBS buffer solutions to remove unreacted DNA. Thereafter, 5 nm gold nanoparticles having positive electric charges were reacted for 30 minutes, washed with PBST and PBS buffer solutions, and then reacted with a gold enhancing solution (Nanoprobes, USA) for 1 minute. Then, the hybridized biochip was washed with water, and the specific hybridization reaction was analyzed.

As a result, gray-colored spots were observed with the naked eye in the region showing the specific hybridization with the target DNA (FIG. 3). FIG. 3 shows photographs of the spots formed on the glass substrate taken by a general scanner. In FIG. 3(a), gray-colored spots were formed by specific binding of the target DNA with the probe H5. In FIG. 3(b), gray-colored spots were formed by specific binding of the control DNA with the control HN.

The spots were not observed or were weakly observed in the non-complementary probe PNA and target DNA. Thus, the results showed that the hybridization reaction of the present invention specifically occurs, gold nanoparticles bind to the hybridized probe PNA and target DNA by electrostatic attraction, and treatment of the gold enhancing solution inducing reduction reaction increases the particle size by reduction of the metal ions using the gold nanoparticles as a catalyst, thereby detecting the spots with the naked eye without additional optical system.

Quantification analysis of the spots obtained at each concentration of the target DNA was performed with grayscale values using Adobe Photoshop software (FIG. 4). As shown in the graph, the grayscale values were found to increase depending on the concentration from 1 pM to 100 nM. In contrast, background staining was observed in the surrounding substrate that did not bind with the target, irrespective of the concentration.

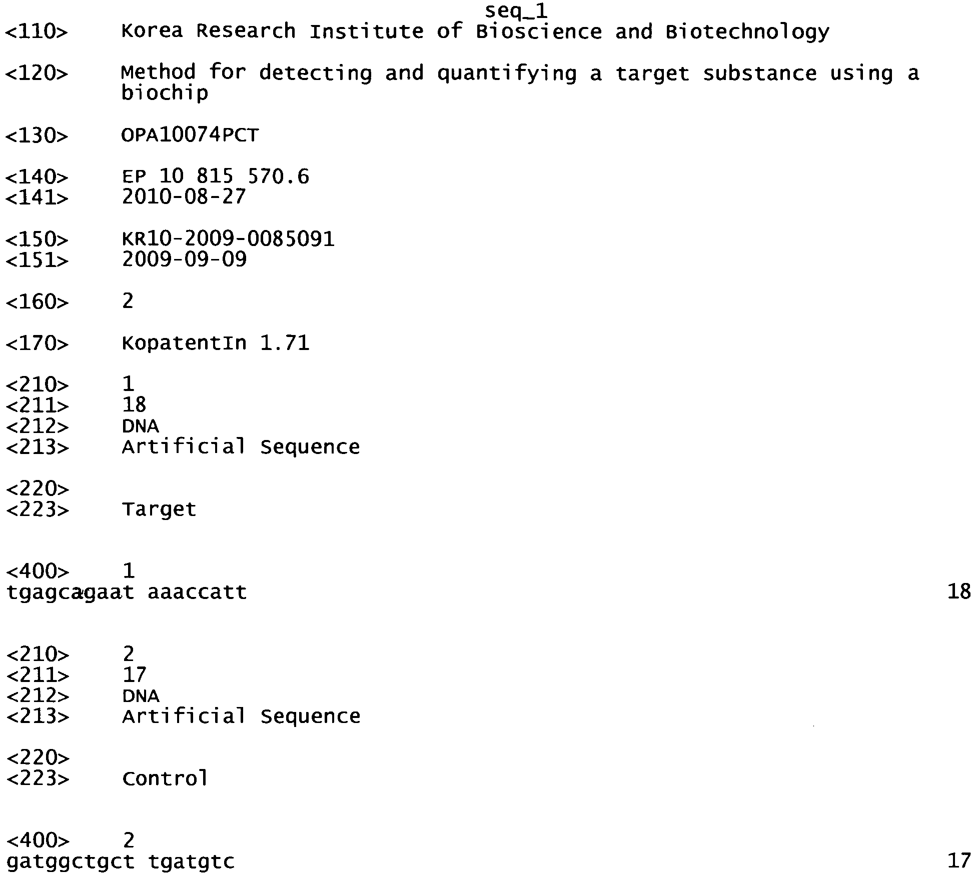

## Claims

1. A method for detecting a target substance using a biochip, comprising the steps of:
(a) fixing probe molecules onto a substrate;
(b) contacting the substrate, onto which probe molecules are fixed, with a sample containing the target substance having electric charges;
(c) reacting the target substance with nanoparticles having electric charges that are opposite to those of the target substance; and
(d) reacting with a metal enhancing solution.

2. A method for quantifying a target substance using a biochip, comprising the steps of:
(a) fixing probe molecules onto a substrate;
(b) contacting the substrate, onto which probe molecules are fixed, with a sample containing the target substance having electric charges;
(c) reacting the target substance with nanoparticles having electric charges that are opposite to those of the target substance;
(d) reacting with a metal enhancing solution; and
(e) measuring the reaction intensity of the region that reacts with the metal enhancing solution.

3. The method according to claim 1, wherein the method for detecting a target substance is performed with the naked eye.

4. The method according to claim 1 or 2, wherein the substrate of step (a) is any one selected from the group consisting of glass, alumina, ceramic, carbon, gold, silver, copper, aluminum, and silicon.

5. The method according to claim 1 or 2, wherein the probe molecule of step (a) is any one selected from the group consisting of PNA (peptide nucleic acid), LNA (locked nucleic acid), peptide, polypeptide, protein, RNA, and DNA.

6. The method according to claim 1 or 2, wherein the target substance of step (b) is any one selected from the group consisting of PNA (peptide nucleic acid), LNA (locked nucleic acid), peptide, polypeptide, protein, RNA, and DNA.

7. The method according to claim 1 or 2, wherein the nanoparticle of step (c) is gold (Au), silver (Ag) or platinum (Pt).

8. The method according to claim 1 or 2, wherein the reaction of the nanoparticle of step (c) is performed by electrostatic binding.

9. The method according to claim 1 or 2, wherein the reaction of the nanoparticle of step (c) is performed by biological binding.

10. The method according to claim 1 or 2, wherein the reaction of the nanoparticle of step (c) is performed by chemical binding.

11. The method according to claim 1 or 2, wherein the metal enhancing solution of step (d) amplifies the size of the reacted nanoparticles of step (c) by reduction of metal ions.

12. The method according to claim 1 or 2, wherein the metal enhancing solution of step (d) includes gold (Au), silver (Ag), copper (Cu), platinum (Pt), or palladium (Pd) ions.
